Europäisches Patentamt

European Patent Office

Office européen des brevets

(11) Publication number: **0 180 192 B1**

# EUROPEAN PATENT SPECIFICATION

(45) Date of publication of patent specification: **05.02.92**

(51) Int. Cl.5: **C12N 15/60**, C12N 15/68, C12N 15/69, C12N 1/20, C12P 13/22, //C12R1/19, C12R1/185

(21) Application number: **85113730.7**

(22) Date of filing: **29.10.85**

(54) **Novel plasmids.**

(30) Priority: **29.10.84 JP 225906/84**
**21.01.85 JP 7450/85**

(43) Date of publication of application:
**07.05.86 Bulletin 86/19**

(45) Publication of the grant of the patent:
**05.02.92 Bulletin 92/06**

(84) Designated Contracting States:
**DE FR GB**

(56) References cited:
**EP-A- 0 080 378**
**EP-A- 0 096 586**

**Kahn, M et al (1979) Methods in enzymology, Vol. 68, pages 268-280**

**Patent abstracts of Japan, unexamined applications, C Field, vol. 2, n 108, May 11 1985**

(73) Proprietor: **RESEARCH ASSOCIATION FOR UTILIZATION OF LIGHT OIL**
**4-11, Shiba 3-chome Minato-ku Tokyo(JP)**

(72) Inventor: **Yukawa, Hideaki**
**576-81, Wakaguri Ami-machi Inashiki-gun Ibaraki-ken(JP)**
Inventor: **Terasawa, Masato**
**Shinwakaguriryo 1315, Oaza Wakaguri Ami-machi Inashiki-gun Ibaraki-ken(JP)**
Inventor: **Kurusu, Yasuro**
**Shinwakaguriryo 1315, Oaza Wakaguri Ami-machi Inashiki-gun Ibaraki-ken(JP)**
Inventor: **Shimazu, Mitsunobu**
**Shinwakaguriryo 1315, Oaza Wakaguri Ami-machi Inashiki-gun Ibaraki-ken(JP)**

(74) Representative: **Kraus, Walter, Dr. et al**
**Patentanwälte Kraus, Weisert & Partner Thomas-Wimmer-Ring 15 W-8000 München 22(DE)**

EP 0 180 192 B1

The Journal of general microbiology, vol. 118, 1980 (Cambridge, Mass.) T. Imanaka et al. "Phenotypic stability of trp Operon re-combinant plasmds in Escherichia coli". pages 254-261

## Description

This invention relates to a novel plasmid containing a DNA fragment comprising genes which govern the biosynthesis of tryptophan synthase. More specifically, it relates to a plasmid containing a DNA fragment comprising genes which govern the biosynthesis of tryptophan synthase, for example tryptophan operon or a DNA fragment at least comprising trp A and trp B and a DNA fragment having a promoter function of expressing trp A and trp B. The novel plasmid has a large copy number and excellent stability in host cells, and in cell proliferation, is exactly partitioned between daughter cells without deletion.

Extensive reasearch has been done heretofore on the cloning of DNA fragments containing genes which govern the biosynthesis of tryptophan synthase. However, such DNA fragments have unsatisfactory stability in host cells as described, for example in Journal of General Microbiology, Vol. 118, 253 (1980), and their application to industrial production of tryptophan is considered to encounter many difficulties.

In Methods in Enzymology, Vol. 68, pages 268-280 a plasmid vector (pDF42) is described comprising a fragment containing genes which are derived from F-plasmid (mini-F) and its partition system, a DNA fragment containing genes which are derived from a ColE1-type plasmid and governs its autonomous replication and a DNA fragment having a promoter (trp-promoter) function of expression an inserted structural gene, and a DNA fragment having an operator function of controlling said promoter function. If pDF42, however, is replicated in the presence of chloramphenicol, reproduction (amplification) of the plasmid DNA occurs suddenly and no propagation of bacterial cells takes place at all.

On the other hand, the most important problem in practice in the stability of a plasmid in host cells is generally the phenomenon of deletion of the plasmid in the subcultivation of the host cells. Various attempts have therefore been made to prevent deletion of plasmids. For example, a method for stabilizing the properties of a microorganism containing a plasmid was proposed in which a plasmid having inserted thereinto a DNA fragment containing a chromosomal gene which governs the property of a microorganism of the genus Escherichia to be independent on streptomycin is included into a streptomycin-dependent mutant of the genus Escherichia (US-A-4,371,615). This method, however, is economically disadvantageous. Moreover, since complex functions need to be incorporated in the desired plasmid, it is anticipated that the plasmid will not easily be partitioned stably between daughter cells during proliferation of the host. Industrial application of this method would be considerably difficult.

With the foregoing background, the present inventors extensively worked for a plasmid having the function of stably partitioning a DNA fragment containing genes governing the biosynthesis of tryptophan synthase, such as tryptophan operon, in a parent cell between daughter cells in subcultivation, and noticed that a DNA fragment containing genes which govern the partition system of F-plasmid contributes greatly to the stabilization of plasmids. On this basis, the present inventors previously created and proposed a novel plasmid, named "plasmid pMTY-1" by the inventors, which comprises a DNA fragment containing genes governing the biosynthesis of tryptophan synthase and a DNA fragment containing genes governing the partition system of the F-plasmid (Japanese Laid-Open Patent Publication No. 2189/1985). This new plasmid pMTY-1 is useful for expressing a gene unstable in host cells, but has the disadvantage that its copy number is as small as 1 to 2, and it has a low efficiency in obtaining large amounts of the product of the gene within the cells.

It has been desired therefore to develop a plasmid which has a large copy number and a high expression efficiency, is stable, and is exactly partitioned between daughter cells in subcultivation with little deletion in the cells of a transformed microorganism.

The present inventors have noted that a Col El-type plasmid of E. coli usually has a copy number of several tens per chromosome, and have succeeded in creating a plasmid which has a large copy number and can be stably partitioned in subcultivation by combining a DNA fragment containing genes which are derived from the Col El-type plasmid and govern its autonomous replication, a DNA fragment containing genes which are derived from the F-plasmid and govern its partition system and a DNA fragment containing genes which govern the biosynthesis of tryptophan synthase.

According to this invention, there is provided a novel plasmid which can be autonomously replicated within the cells of Escherichia coli, in which plasmid

(a) a DNA fragment containing genes which govern the biosynthesis of tryptophan synthase consists at least of trp A, trp B, a DNA fragment having a promoter function of expressing said two genes, and a DNA fragment having an operator function of controlling said promoter function,

(b) a DNA fragment containing genes which are derived from F-plasmid and govern its partition system is a mini-F fragment,

(c) a DNA fragment containing genes which are derived from the Col El-type plasmid and govern its autonomous replication is derived from plasmid pBR322,

EP 0 180 192 B1

(i) the direction of transcription from the promoter in the DNA fragment containing genes governing the biosynthesis of tryptophan synthase, the direction of replication from the replication initiation point of the mini-F fragment, and the direction of replication from the replication initiation point of the DNA fragment containing genes which are derived from Col El-type plasmid and govern its autonomous replication are identical, and

(ii) the DNA fragment containing genes governing the biosynthesis of tryptophan synthase is located upstream of the DNA fragment containing genes which are derived from the Col El-type plasmid and govern its autonomous replication.

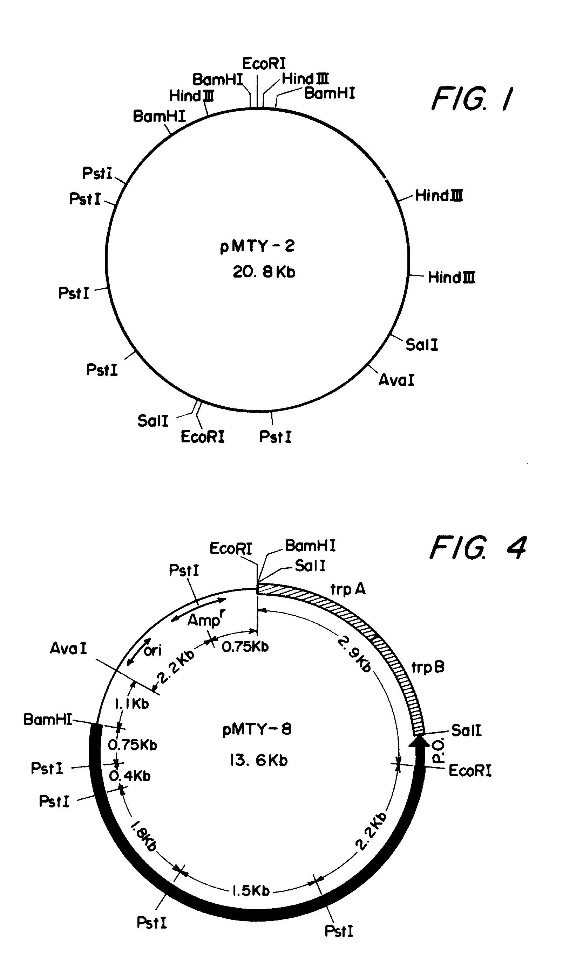

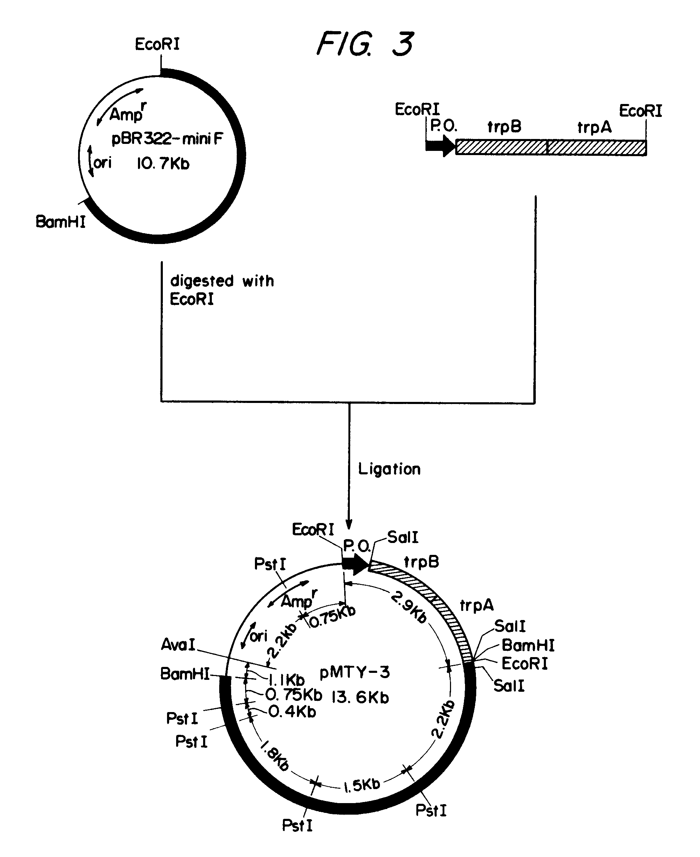

This invention refers further to a plasmid which is plasmid pMTY-2 according to Figure 1 having a molecular size of about 13.7 megadaltons and 2, 3 and 2 sites of recognition by restriction endonucleases EcoRI, BamHI and SalI, respectively and to a plasmid which is plasmid pMTY-3 according to Figure 3 having a molecular size of about 9.0 megadaltons and 2, 2 and 3 sites of recognition by restriction endonucleases EcoRI, BamHI and SalI, respectively.

The "DNA fragment containing genes which govern the biosynthesis of tryptophan synthase" (to be sometimes abbreviated as "T fragment") constituting the plasmid of the invention denotes a DNA fragment containing genes which govern the biosynthesis of L-tryptophan from indole and L- or DL-serine. The T fragment includes, for example, tryptophan operon, a larger DNA fragment containing tryptophan operon, a DNA fragment containing trp A and trp B, which are genes included within tryptophan operon, and governing the biosynthesis of tryptophan synthase, and a promoter and an operator joined thereto, and a DNA fragment containing trp A and trp B and at least one of trp C, trp D and trp E, and a promoter and an operator jointed thereto (for example a DNA fragment containing trp A, trp B, trp C, trp E, a promoter and an operator).

If a microorganism transformed with the plasmid of this invention is used, the production of tryptophan by a so-called fermentation method becomes economically feasible. In the production of tryptophan from indole and L-serine (in actual operation, DL-serine may be added to the reaction system instead of L-serine because only L-serine reacts selectively) as materials by utilizing only the tryptophan synthase action derived from tryptophan operon, the efficiency of biosynthesis of tryptophan decreases because tryptophan operon as the T fragment has the function of jointly producing three other enzymes in addition to tryptophan synthase.

In order to increase the efficiency of biosynthesis of tryptophan synthase in tryptophan operon, the present inventors extensively worked on a plasmid which meets this purpose. Consequently, the present inventors have found that a plasmid comprising a combination of a DNA fragment which is obtained by ligating a DNA fragment having a promoter function of expressing trp A and trp B and optionally a DNA fragment having an operator function of controlling the promoter function to trp A and trp B fragments which are genes governing biosynthesis of tryptophan synthase, a DNA fragment containing genes which are derived from the F-plasmid and govern its partition system and a DNA fragment containing genes which are derived from the Col El-type plasmid and govern its autonomous replication markedly increases the efficiency of producing tryptophan synthase.

Accordingly, the T fragment used in this invention is preferably a DNA fragment obtained by ligating a DNA fragment having a promoter function of expressing trp A and trp B (to be sometimes referred to as "tryptophan promoter") and as required, a DNA fragment having an operator function of controlling the promoter function (to be sometimes referred to as "tryptophan operator") to trp A and trp B fragments. In practice, the T fragment is preferably that derived from E. coli. There is no particular restriction on the source of the T fragment, but E. coli ATCC 23282, E. coli ATCC 23437 and E. coli ATCC 23461 can be advantageously used.

A detailed method of preparing the T fragment suitable for the purpose of this invention from these bacteria, for example a tryptophan operon or a DNA fragment composed of trp A and trp B fragments, a tryptophan promoter and a tryptophan operator is shown in Example 1, (C) and Example 3, (B) given hereinbelow. The basic procedure comprises infecting phage $\phi$ 80 with an E. coli strain having tryptophan operon in its chromosomal genes, and then inducing the phage to prepare a large amount of the phage having tryptophan operon incorporated in the phage DNA. Then, the phage DNA is extracted and a tryptophan operon DNA fragment is cut out from it by using restriction endonucleases BamHI, EcoRI, etc. As required, this DNA fragment is further partially cleaved with restriction endonuclease HincII to obtain a DNA fragment containing trp A and trp B genes. A DNA fragment containing a promoter and an operator can be obtained by treating the tryptophan operon DNA fragment with restriction endonuclease PvuII. The resulting two DNA fragments are ligated by using a DNA ligase to prepare a DNA fragment composed of trp A and trp B fragments and the tryptophan promoter and the tryptophan operator joined thereto.

One characteristic feature of this invention is that as described hereinabove, the T fragment is combined with the DNA fragment containing genes which are derived from F-plasmid and govern its partition system. The F-plasmid is a known plasmid having a molecular size of 94.5 Kb ($62 \times 10^6$ daltons) having the structure shown in the genetic map and the physical map by EcoRI shown at page 64 of R. A. Skurray, H. Nagaishi and A. J. Clark: Proc. Natl. Acad. SCi., U.S.A., Vol. 73, page 64 (1976). The F-factor plasmid exists in enteric bacteria such as E. coli usually in a copy number of 1 to 2 per cell chromosome. This plasmid has a mechanism whereby it is accurately transmitted to daughter cells after cell division (the proliferation accurately in accord with the pace of proliferation of a host in spite of a low copy number level as in this case is called the "stringent" control of proliferation). It has already been found that such a function of stringently controlling proliferation in the F-plasmid is carried on a fragment, called mini-F, which has a molecular size of 9.1 Kb and can replicate autonomously. It is also known that the mini-F can be cut out from the F-plasmid by restriction endonuclease EcoRI

The present invention utilizes the partition system of the mini-F. Thus, the "DNA fragment containing genes which are derived from the F-plasmid and govern its partition system" (to be sometimes abbreviated as the "F fragment") denotes a DNA fragment having a mechanism of accurately transmitting the F-plasmid to daughter cells". This F fragment is a mini-F fragment having a molecular size of about 9.1 Kb.

The "DNA fragment containing genes which are derived from a Col El-type plasmid and which govern its autonomous replication" (to be sometimes abbreviated as "S fragment") to be used in combination with the T and F fragments in this invention denotes a DNA fragment which has a copy number of 20 to 30 per cell chromosome and contains genes governing the autonomous replication of the Col El-type plasmid. This S fragment is derived from the plasmid pBR322 and has a size of about 4.3 Kb.

The plasmid provided by this invention comprises the T fragment, F fragment and S fragment as essential constituents, and may further include DNA fragments bearing other genetic information, for example a DNA fragment containing an ampicillin-resistance gene which is an antibiotic resistance marker, and a DNA fragment containing a kanamycin-resistance gene

One typical example of the plasmid provided by this invention is a plasmid, named "plasmid pMTY-2" by the present inventors, which consists essentially of the T fragment (tryptophan operon DNA fragment), the F fragment (mini-F fragment) and the S fragment and having a molecular size of about 13.7 megadaltons (about 20.8 Kb).

In the present application, the molecular sizes of plasmids are determined by the agarose gel electrophoretic method.

The plasmid pMTY-2 will be described in detail.

The sensitivities (the number of recognition or cleavage sites) of the plasmid pMTY-2 to the following restriction endonucleases, and the lengths (Kb) of fragments cleaved with these restriction endonucleases are tabulated below.

| Restriction endonuclease | Number of recognition sites | Length of the fragment (Kb) |
|---|---|---|
| EcoRI | 2 | 11.7, 9.1 |
| BamHI | 3 | 17.8, 2.4, 0.6 |
| SalI | 2 | 17.0, 3.8 |
| AvaI | 1 | 20.8 |
| HindIII | 4 | 14.4, 3.0, 2.6, 0.8 |
| PstI | 5 | 14.1, 3.0, 1.8, 1.5, 0.4 |

The plasmid pMTY-2 having the above characteristics can be prepared, for example, by the following procedure.

First, the tryptophan operon DNA fragment (T fragment) is prepared by, for example, infecting a phage, such as phage φ 80 (ATCC 11456a-Bl) with E. coli having tryptophan operon in its chromosomal gene, such as Escherichia coli K-12 (IFO 3301, ATCC 10798, or ATCC e23562), lyzing and inducing the phage to prepare a large amount of a phage having tryptophan operon incorporated in the phage DNA [see R. M.

Denney, C. Yanofsky: J. Bacteriol., 118, 505 (1974)1, extracting the phage DNA from it [T. Maniatis, E. F. Fritsch, J. Sambrook: "Molecular Cloning" (1982), pages 164-165, Cold Spring Harbor Laboratory], and cutting out the tryptophan operon from the extracted phage DNA using a restriction endonuclease such as BamHI or EcoRI.

The mini-F fragment may be prepared, for example, by extracting the F-plasmid from a microorganism harboring the F-plasmid, such as E. coli K-12 (ATCC 15153, ATCC e23589, or ATCC e23590), by a method known per se , for example by the method described in P. Guerry, D. L. LeBlanc, S. Falkow: J. Bacteriol, 116, 1064 (1973), and cutting out a mini-F fragment having a molecular size of about 9.1 Kb from the F-plasmid using restriction endonuclease EcoRI.

It is convenient to use plasmid pBR322 which is a typical example of the Col El plasmid as a source of the fragment containing genes governing the autonomous replication of the Col El plasmid.

The tryptophan operon DNA fragment (T fragment) prepared as above and plasmid PBR322 treated with the same restriction endonuclease as that used in cutting out the T fragment were ligated by means of a ligase such as ligase derived from T4 phage to prepare a plasmid having the T fragment inserted into the plasmid pBR322. The resulting plasmid is then cleaved with restriction endonuclease EcoRI, and ligated with the mini-F fragment prepared as above by the action of a ligase. As a result, the desired plasmid pMTY-2 is obtained.

The preparation of the plasmid pMTY-2 is more specifically described in Example 1 below.

The plasmid pMTY-2 of this invention so prepared has a large copy number, is stable and is not deleted when partitioned between daughter cells during the cell division of the host.

Another typical example of the plasmid provided by this invention is a plasmid, named "plasmid pMTY-3" by the present inventors, which consists essentially of the T fragment (trpA + trp B + tryptophan promoter + tryptophan operator), the F fragment (mini-F fragment) and the S fragment and has a molecular size of about 9.0 megadaltons (about 13.6 Kb).

The plasmid pMTY-3 will be described in detail below.

The sensitivities (the number of recognition sites) of the plasmid pMTY-3 to the following restriction endonucleases, and the lengths (Kb) of fragments cleaved with these restriction endonucleases are tabulated below.

| Restriction endonuclease | Number of recognition site | Length of the fragment (Kb) |
|---|---|---|
| EcoRI | 2 | 10.7, 2.9 |
| BamHI | 2 | 6.9, 6.7 |
| SalI | 3 | 10.6, 2.9, 0.1 |
| PstI | 5 | 5.9, 4.0, 1.8, 1.5, 0.4 |
| AvaI | 1 | 13.6 |

The plasmid pMTY-3 having the above characteristics can be produced, for example, by the following procedure.

First, the T fragment consisting of trp A, trp B, tryptophan promoter and tryptophan operator is prepared as follows: A phage, such as phage $\phi$ 80 (ATCC 11456a-Bl), is infected with E. coli having tryptophan operon in chromosomal genes, such as E. coli K-12 (IFO 3301, ATCC 10798 or ATCC e23562), and lyzing and inducing the phage to prepare a large quantity of a phage having tryptophan operon incorporated in the phage DNA [see R. M. Denney, C. Yanofsky: J. Bacteriol., 118, 505 (1974)], and extracting the phage DNA from it [see T. Maniatis, E. F. Fritsch, and J. Sambrook: "Molecular Cloning" (1982), pages 164-165, Cold Spring Harbor Laboratory], cutting out a tryptophan operon DNA fragment from it using a restriction endonuclease such as BamHI and EcoRI, and further partially cleaving the DNA fraction with restriction enzyme HincII to obtain a DNA fragment containing trp A and trp B genes. Treatment of the tryptophan operon DNA fragment with restriction endonuclease PvuII gives a DNA fragment containing a promoter and an operator. The resulting two DNA fragments are ligated using T4 DNA ligase derived from T4 phage. As a result, a T fragment is obtained in which the DNA fragment containing the promoter and the operator is ligated with trp A and trp B fragments and which contains an EcoRI site at both terminals.

The mini-F fragment may be prepared, for example, by extracting the F-plasmid from a microorganism harboring the F-plasmid, such as E. coli K-12 (ATCC 15153, ATCC e23589, or ATCC e23590), by a method known per se , for example by the method described in P. Guerry, D. L. LeBlanc, S. Falkow: J. Bacteriol, 116, 1064 (1973), and cutting out a mini-F fragment having a molecular size of about 9.1 Kb from the F-plasmid using restriction endonuclease EcoRI.

As a source of the fragment containing genes governing the autonomous replication of the Col EI plasmid, it is convenient to use plasmid pBR322 which is a typical example of Col EI plasmid.

The mini-F fragment prepared as above is treated with restriction endonucleases EcoRI and BamHI is ligated by means of T4 DNA ligase with plasmid pBR322 treated with the same restriction endonucleases to construct a plasmid having the mini-F fragment inserted into plasmid pBR322. The resulting recombinant plasmid is cleaved with EcoRI, and ligated with the T fragment prepared as above by the action of T4 DNA ligase to thereby obtain the desired plasmid pMTY-3.

The preparation of the plasmid pMTY-3 will be more specifically described in Example 3 given hereinbelow.

The plasmid pMTY-3 of this invention so prepared has a large copy number, is stable and is not deleted when partitioned between daughter cells during the cell division of the host.

Still another typical example of the plasmid provided by this invention is a plasmid, named "plasmid pMTY-8" by the present inventors, which consists substantially of the T fragment, F fragment and S fragment and has a molecular size of about 9.0 megadaltons (about 13.6 Kb), wherein

    (a) the T fragment consists at least of trp A, trp B, a tryptophan promoter and a tryptophan operator,

    (b) the F fragment is a mini-F fragment,

    (c) the S fragment is derived from plasmid pBR322,

        (i) the direction of transcription from the promoter in the DNA fragment containing genes governing the biosynthesis of tryptophan synthase, the direction of replication from the replication initiation point of the mini-F fragment, and the direction of replication from the replication initiation point of the DNA fragment containing genes which are derived from Col EI-type plasmid and govern its autonomous replication are identical, and

        (ii) the DNA fragment containing genes governing the biosynthesis of tryptophan synthase is located upstream of the DNA fragment containing genes which are derived from the Col EI-type plasmid and govern its autonomous replication.

The plasmid pMTY-8 will be described in detail below.

The sensitivities (the number of recognition sites) of the plasmid pMTY-8 to the following restriction endonucleases, and the lengths (Kb) of fragments cleaved with these restriction endonucleases are tabulated below.

| Restriction endonuclease | Number of recognition sites | Length of the fragment (Kb) |
|---|---|---|
| EcoRI | 2 | 10.7, 2.9 |
| BamHI | 2 | 9.7, 3.9 |
| SalI | 3 | 10.6, 2.9, 0.1 |
| PstI | 5 | 5.9, 4.0, 1.8, 1.5, 0.4 |
| AvaI | 1 | 13.6 |

The plasmid pMTY-8 having the above characteristics can be produced, for example, by the following procedure.

A detailed method for preparing plasmid pMTY8 is shown in Example 6 given hereinbelow. The basic procedure is as follows: A DNA fragment containing genes governing the biosynthesis of tryptophan synthase is cut out from pMTY-3 using restriction endonuclease EcoRI. pMTY-3 is deposited as introduced into E. coli K12 YK2009 (Deposit No. FERMP-7957) and can be extracted from the cultivated cells [see T. Maniatis, E. F. Fritsch and J. Sambrook: "Molecular Cloning" (1982 ), pages 86-94]. The DNA fragment so cut out was re-ligated using T4 DNA ligase derived from T4 phage. As a result, the plasmid pMTY-8 is constructed in a proportion of about 10% based on the plasmid pMTY-3.

The plasmid pMTY-8 of this invention so prepared, like the plasmid pMTY-3, has a large copy number, is stable and is not deleted when partitioned between daughter cells during the cell fission of the host.

The plasmids of this invention are much expected to be applicable to the industrial production of tryptophan.

In the production of tryptophan, a host organism is transformed with the plasmid of the invention. Possible candidates for such a host organism include E. coli, B. subtilis and Saccharomyces strains. Microorganisms of the genus Escherichia, particularly microorganisms of the E. coli K-12 type, are preferred. Of these, strains having no activity of decomposing L-tryptophan (i.e., tryptophanase-deficient mutants), are especially preferred.

The plasmid of this invention can be introduced into the host organism by a method known per se, for example the method described in M. Mandel and A. Higa: J. Mol. Biol. 53, 159 (1970).

The host organism so transformed is cultivated by a method known per se,to produce and accumulate tryptophan synthase fully within the cells, and then can be utilized in an enzymatic reaction for the production of L-tryptophan from indole and L- or DL-serine.

The cultivation of the transformed host organism can be carried out by a method known per se. Culture media that can be used may include any natural and synthetic media containing carbon sources, nitrogen sources and inorganic salts which are used in ordinary microorganism cultivation. Examples of the carbon sources are various carbohydrates such as glucose, glycerol, fructose, sucrose and molasses. Examples of the nitrogen sources are natural organic nitrogen sources such as tryptone, yeast extract, corn steep liquor and casein hydrolyzate. Many of the natural organic nitrogen sources can also serve as carbon sources. Examples of the inorganic salts include potassium monohydrogen phosphate, potassium dihydrogen phosphate, magnesium sulfate, sodium chloride, ferrous sulfate, calcium chloride and manganese chloride.

Other nutrients such as vitamins and amino acids useful for the growth of microorganisms may be added to the culture media.

Furthermore, indoleacrylic acid or its salt may be added to the culture media. This addition has been found to markedly increase the amount of tryptophan synthase produced. Indoleacrylic acid or its salt may be added in the initial stage of cultivation or during the cultivation. In the latter case, it is desirable to add it at the latest before the final stage of the logarithmic growth period of the microorganism. The addition may be performed once or several times, or continuously.

The amount of indoleacrylic acid or its salt added is not strictly limited. Generally, it may be added in such an amount that the final concentration of it based on the culture medium is at least 25 micrograms/ml, preferably 80 to 100 micrograms/ml, more preferably 100 to 200 micrograms/ml.

The salt of indoleacrylic acid may be an alkali metal indoleacrylate such as sodium indoleacrylate.

The "final concentration", as used herein, denotes the total concentration of indoleacrylic acid or its salt added to the culture medium until the end of the cultivation.

In the process of this invention, it is desirable to use glucose as at least part of the carbon source. The amount of glucose used is generally 10 to 600 moles, preferably 50 to 400 moles, more preferably 100 to 300 moles, per mole of indoleacrylic acid or its salt.

The cultivation time varies depending upon the type of the microorganism, the other cultivation conditions, etc. Usually, it is about 1 to about 120 hours. The cultivation temperature is usually about 20 to about 50 °C, and the pH of the culture medium is generally 5 to 9, preferably about 6 to about 8.

The cultivation is preferably carried out under aerobic conditions by shaking, or by aeration and agitation.

The microorganism cells so cultivated may be directly utilized in the enzymatic reaction of producing L-tryptophan. They may, however, be used in the form of a crushed product obtained by crushing the cells by, for example, ultrasonication, an extract obtained by extracting the crushed product with water or the like, or a partially purified product obtained by treating the extract with ammonium sulfate, etc. to precipitate the enzyme component. As required, the cells and their treated products may be immobilized prior to use.

The reaction of indole with L- or DL-serine in the presence of the above cells or their treated product is carried out, as in an ordinary enzymatic reaction, in a solvent such as 0.1M phosphate buffer (pH 7.0 - 9.0) or water (pH 7.0 - 9.0) at a temperature of about 20 to about 50 °C, preferably about 30 to about 40 °C, usually for a period of about 0.1 to about 72 hours.

The amounts of indole and L- or DL-serine used at the time of the reaction are not particularly restricted. Generally, these materials are suitably used each in a concentration of 0.01 to 20 % (wt/vol). The amount of the cells or the treated product thereof used are neither restricted in particular, and may generally be 0.1 to 10 % (wt/vol).

After the reaction, L-tryptophan may be separated from the reaction mixture and purified by methods known per se, for example adsorption and desorption using an ion-exchange resin or activated carbon.

The enzymatic process described above is very advantageous industrially because it can markedly increases the rate of production of tryptophan in the production of L-tryptophan by reacting indole and L- or DL-serine in the presence of the cultivated cells of a microorganism of the genus Escherichia transformed with the plasmid provided by this invention, for example the plasmid pMTY-2, pMTY-3 or pMTY-8, or a product obtained by treating the microbial cells.

The host organism transformed with the plasmid of this invention may also be utilized in the production of L-tryptophan by a fermentation method. Specifically, when the transformed host is cultivated, L-tryptophan is produced and accumulated in the culture medium and can be recovered from the culture broth.

The cultivation can be carried out by using the same medium as described above under the same conditions as described above. In the fermentation method, indole or anthranilic acid or its salt (the salt may be the same as exemplified above with regard to the salt of indoleacrylic acid) may, as required, be added to the culture medium as a precursor of L-tryptophan. In the case of cultivating E. coli K-12 YK2004 transformed with the plasmid pMTY-2, it is prefered to add indole or anthranilic acid or its salt. In the case of E. coli K-12 YK2009 transformed with pMTY-3 or E. coli K-12 YK2014 transformed with pMTY-8, the addition of indole is preferred. The addition may be effected in the initial stage of the cultivation or during the cultivation as in the case of adding indoleacrylic acid or its salt. When the addition is effected during the cultivation, it should desirably be done at the latest before the final stage of the logarithmic growth period of the microorganism. The addition may be carried out once or a plurality of times or continuously. The amount of indole or anthranilic acid or its salt is not strictly limited. For example, when glucose is used as the carbon source, the amount of indole or anthranilic acid or its salt is 0.005 to 1 mole, preferably 0.01 to 0.5 mole per mole of glucose.

L-Tryptophan may be recovered from the culture broth obtained by cultivation under the above cultivation conditions by methods known per se, for example by adsorption-desorption treatment using an ion-exchange resin, activated carbon, etc.

The process of the invention described above can give L-tryptophan very efficiently, and is very advantageous industrially.

A plasmid containing the DNA fragment containing genes governing the partition system of the F-plasmid (such as the mini-F fragment), the DNA fragment containing genes which are derived from Col EI plasmid (such as pBR322) and govern its autonomous replication, and a promoter capable of expressing a foreign gene (such as a promoter derived from tryptophan operon), and preferably further containing an operator can be used as a vector. An example of the foreign gene used is a structural gene corresponding to tryptophan synthase.

The following Examples illustrate the present invention more specifically.

EXAMPLE 1

Construction of plasmid pMTY-2

(A) Preparation of a mini-F fragment

E. coli K-12 strain (ATCC 15153) was inoculated in one liter of L medium (Bacto tryptone 10 g, yeast extract 5 g, NaCl 5 g, glucose 1 g, water 1 liter; pH 7.2), and cultivated with shaking at about 37°C for 4 hours. The cells were harvested, treated with lysozyme, and lyzed with sodium dodecylsulfate (SDS). The lysate was centrifuged for 40 minutes at 32,000 x G. The supernatant was separated, subjected to caesium chloride-ethidium bromide equilibrium density gradient centrifugation and then dialyzed to separate a solution containing the F-plasmid. The solution was precipitated with ethanol to finally collect about 20 micrograms of the F-plasmid.

Then, 5 micrograms of the F-plasmid was taken and subjected to the action of restriction endonuclease EcoRI for 1 hour at 37°C to cleave the DNA. As a result, a DNA solution containing a mini-F fragment having a size of about 9.1 Kb was prepared.

(B) Preparation of phage φ 80pt

E. coli K-12 strain (IFO 3301) was inoculated in 100 ml of L medium (having the same composition as indicated above), and cultivated with shaking at 37°C for 4 hours. 0.5 mg of the culture broth was mixed with 0.1 ml of an aqueous solution of phage $\phi$ 80 (ATCC 11456a-B$_1$) ($10^5$/ml) in L medium soft agar (L medium + agar powder), and the mixture was overlaid on an L medium agar plate. When the plate was cultivated at 37°C for about 5 hours, a plaque formed. When the cultivation was continued further for 2 to 3 days at 37°C, grown colonies of phage $\phi$ 80 lysogenic bacteria formed in the plaque. The lysogenic bacteria were cultivated in L medium at 37°C for 4 hours and plated on the same L medium agar plate as above, and then by the induction of the lysogenic phage by ultraviolet radiation (400 - 800 ergs/mm$^2$, 10 - 20 seconds), phage $\phi$ 80 pt (a phage DNA containing tryptophan operon) was prepared.

(C) Preparation of a tryptophan operon DNA fragment

E. coli K-12 strain (IFO 3301) was inoculated in 1 liter of L medium having the same composition as indicated above, and cultivated with shaking at about 37°C for about 3 hours. In the logarithmic growth period, 10 ml of a 25% (w/v) glucose solution and the phage $\phi$ 80 pt solution prepared in (B) were added (moi 20) in a concentration of $10^{11}$/ml. The shaking culture was continued for 5 hours, and by the addition of chloroform, a large amount of phage $\phi$ 80 pt was prepared [see T. Maniatis, E. F. Fritsch, J. Sambrook: "Molecular Cloning" (1982), pages 76-80, Cold Spring Harbor Laboratory].

The phage $\phi$ 80 pt solution obtained was dialyzed against Tris buffer (pH 7.8), and by the DNA extracting method using phenol (see page 85 of the above-cited "Molecular Cloning"), the phage DNA was recovered and purified. The phage DNA was reacted with restriction endonuclease BamHI at 30 °C for 30 minutes to obtain a tryptophan operon DNA fragment.

(D) Construction of plasmid pMTY-2

The tryptophan operon DNA fragment obtained in (C) was digested with restriction endonucleases SalI and XhoI at 37 °C for 1 hour. The digestion product was then heat-treated at 65 °C for 5 minutes to inactivate the restriction endonucleases, and mixed with plasmid pBR322 treated similarly with restriction endonuclease SalI. ATP (adenosine triphosphate) and DTT (dithiothreitol) were added, and thereafter, DNA ligase derived from T4 phage ("T4 DNA ligase" produced by Takara Shuzo Co., Ltd.) was added. The mixture was ligated at 12 °C for 24 hours, and then heat-treated at 65 °C for 5 minutes to inactivate the DNA ligase. E. coli K-12 strain (tryptophan-auxotrophic mutant) was transformed with the ligated DNA (S. N. Cohen: Proc. Natl. Acad. Sci., 69, 2110-2114, 1972) to obtain transformants [the disappearance of trp auxotrophy; they permit biosynthesis of tryptophan by tryptophan operon on the plasmid and can be grown on a minimum medium (K$_2$HPO$_4$ 7 g, KH$_2$PO$_4$ 2g, MgSO$_4$.7H$_2$O 0.1 g, (NH$_4$)$_2$SO$_4$ 1g, glucose 2 g, pure water 1 liter)]. The transformants were subjected to liquid cultivation, and a plasmid containing tryptophan operon was isolated from the culture broth.

The plasmid containing tryptophan operon was reacted with restriction endonuclease EcoRI at 37°C for 1 hour, and then heat-treated at 65°C for 5 minutes to inactivate EcoRI. The mini-F fragment obtained in (a) above was then added, and ATP, DTT and the DNA ligase were further added. The mixture was maintained at 12°C for 24 hours, and then heat-treated at 65°C for 5 minutes to inactivate the DNA ligase. E. coli K-12 strain (tryptophan-auxotrophic mutant) was transformed with the ligated DNA, and then inoculated in L medium (Bacto-tryptone 10 g, yeast extract 5 g, NaCl 5 g, glucose 1 g, water 1 liter; pH 7.2) containing 50 micrograms/ml of ampicillin and cultivated with shaking at 37°C for 18 hours. The cells were collected by centrifugation, washed and then plated on the minimum medium (having the same composition as above) to obtain transformants. The transformants were subjected to liquid cultivation, and a plasmid was isolated from the culture broth.

The resulting plasmid pMTY-2 has the restriction endonuclease map shown in Figure 1 of the attached drawings. The map was prepared by digesting the plasmid pMTY-2 with restriction endonucleases EcoRI, BamHI, SalI, etc. either singly or in combination, and then subjecting the digestion products to agarose gel electrophoresis.

The transformants harboring the plasmid pMTY-2 were deposited in Fermentation Research Institute, Agency of Ministry of International Trade and Industry, at 1-1-3, Higashi, Yatabe-cho, Tsukuba-gun, Ibaraki-ken, Japan on September 7, 1984 (deposit number FERM P-7838).

EXAMPLE 2

Stability of transformed E. coli K-12 YK2004:-

One hundred milliliters of the minimum medium indicated above was put in a 500 ml Erlenmeyer flask,

and sterilized at 120°C for 15 minutes. Then, the transformants obtained in Example 1 were inoculated in the medium and cultivated with shaking at 37°C for 24 hours. One hundred milliliters of L medium was put in a 500 ml Erlenmeyer flask, and sterilized at 120°C for 15 minutes. The cultivated transformants were then inoculated in the L medium at a rate of 50 cells per ml and cultivated with shaking at 37°C for 24 hours. The cells were harvested by centrifugation, and washed. A predetermined amount of the cells was plated on a plate medium prepared as L medium containing 50 micrograms/ml of ampicillin and L medium containing no ampicillin, and cultivated at 37°C for 1 day. The number of grown colonies was counted.

The number of the grown colonies was the same for the two media, and all the colonies grown on the L medium grew on the minimum medium not containing tryptophan. The results demonstrate the high stability of the above plasmid.

EXAMPLE 3

Construction of plasmid pMTY-3

(A) Preparation of phage φ 80 pt

E. coli K-12 strain (IFO 3301) was inoculated in 100 ml of L medium (Bacto-tryptone 10 g, yeast extract 5 g, NaCl 5 g, glucose 1 g, water 1 liter; pH 7.2), and cultivated at 37°C for about 4 hours. 0.2 ml of the culture was mixed with 0.1 ml of an aqueous solution of phage φ 80 (ATCC 11456a-Bl) ($10^5$/ml) in L medium soft agar (L medium + agar powder), and the mixture was overlaid on L medium agar plate. When the plate was cultivated at 37°C for about 5 hours, a plaque formed. On continuing the cultivation further for 2 to 3 days at 37°C, grown colonies of phage φ 80 lysogenic bacteria formed in the plaque. The lysogenic bacteria were cultivated on L medium at 37°C for 4 hours, and then plated on the same L agar plate as indicated above. Thereafter, by inducing the lysogenic phage under ultraviolet irradiation (400 to 800 ergs/mm$^2$, 10 to 20 seconds), phage φ 80 pt (a phage DNA containing tryptophan operon) was prepared.

(B) Preparation of a T fragment comprising trp A and trp B fragments to which a tryptophan promoter DNA fragment and an operator DNA fragment are joined:-

E. coli K-12 strain (IFO 3301) was inoculated in L medium (having the same composition as above) was cultivated with shaking at about 37°C for about 3 hours. In the logarithmic growth period, 10 ml of a 25% (w/v) glucose solution and the phage φ 80pt solution prepared above in a concentration of $10^{11}$ cells/ml were added (moi 20). The shaking cultivation was continued for 5 hours, and then by adding chloroform, a large amount of phage φ 80pt was prepared [see T. Maniatis, E. F. Fritsch and J. Sambrook: "Molecular Cloning" (1982), pages 76-80, Cold Spring Harbor Laboratory].

The phage φ 80pt solution so obtained was dialyzed against Tris buffer (pH 7.8), and the phage DNA was extracted and purified by the DNA extracting method using phenol (see page 85 of the above-cited "Molecular Cloning"), and digested with restriction endonuclease BamHI at 30°C for 30 minutes to obtain a tryptophan operon DNA fragment.

Then, 10 micrograms of the tryptophan operon DNA fragment was digested partially with restriction endonuclease HincII at 37°C for 5 minutes to prepare 2 micrograms of a DNA fragment containing trp A and trp B genes. Furthermore, 20 micrograms of the tryptophan operon DNA fragment was digested with restriction endonuclease PvuII at 37°C for 1 hour to prepare 0.4 microgram of a DNA fragment including a promoter region and an operator region. The two DNA fragments obtained were mixed and ligated at 12°C for 24 hours with T4 DNA ligase. Furthermore, 0.5 microgram of an EcoRI linker was mixed and the mixture was again subjected to ligation at 12°C for 24 hours using T4 DNA ligase. The re-ligated product was digested with restriction endonuclease EcoRI at 37°C for 1 hour to prepare a T fragment comprising trp A and trp B fragments and the promoter and operator DNA fragments joined with the two cohesive termini being EcoRI sites.

(C) Construction of plasmid pMTY-3

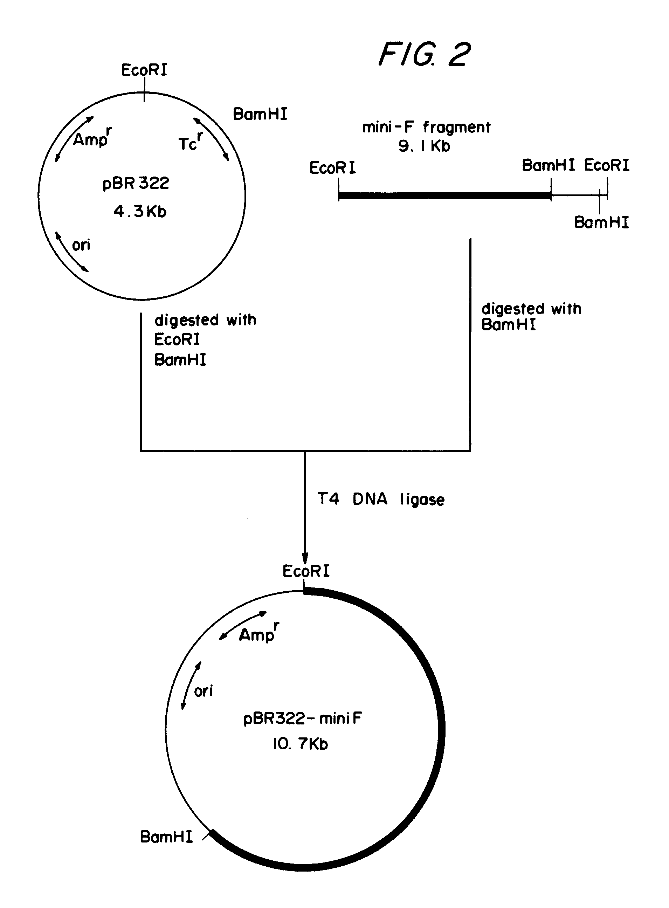

To construct the plasmid pMTY-3 in accordance with this invention, pBR322 mini-F plasmid was first constructed as shown in Figure 2 of the attached drawings.

The mini-F fragment was prepared [T. Mukai, K. Matsubara and Y. Takagi: Proc. Natl. Acad. Sci., U.S.A., 70, 2884 (1973)]. One microgram of the mini-F fragment was digested with restriction endonuclease BamHI at 37°C for 1 hour. Separately, 2 micrograms of plasmid pBR322 DNA having ampicillin resistance (Amp$^r$) and tetracycline resistance (Tc$^r$) was digested with restriction endonucleases EcoRI and BamHI. The two digestion products were mixed and re-ligated at 12°C for 24 hours with T4 DNA ligase. E. coli C600 strain (ATCC e23738) was transformed with the re-ligated DNA, and transformants which have Amp$^r$ and Tc$^s$ (tetracycline sensitivity) were selected. The plasmid DNA was isolated from these transformants and purified. Its molecular size was determined by agarose gel electrophoresis and pBR322-mini F plasmid having a molecular size of about 10.7 Kb was collected.

As shown in Figure 3, pMTY-3 was constructed by ligating the pBR322-mini F plasmid and the T fragment prepared in (B) above.

Specifically, 2 micrograms of pBR322-mini F plasmid DNA was digested with restriction endonuclease EcoRI at 37°C for 1 hour, and 1 microgram of the DNA fragment prepared in (B) above in which the promoter and operator DNA fragments are joined to the trp A and trp B fragments was mixed. They were ligated at 12°C for 24 hours in the presence of T4 DNA ligase. E. coli K-12 strain (tryptophan-auxotrophy mutant; ATCC 23718) was transformed with the re-ligated DNA to obtain transformants [the disappearance of trp auxotrophy; namely, they permit biosynthesis of tryptophan by the trp A and trp B genes on the plasmid and can be grown on the minimum medium ($K_2HPO_4$ 7g, $KH_2PO_4$ 2g, $MgSO_4.7H_2O$ 1g, glucose 2g, pure water 1 liter)]. The transformants were subjected to liquid cultivation, and plasmid pMTY-3 having the restriction endonuclease map of Figure 3 was isolated from the culture broth.

The transformants harboring the plasmid pMTY-3 were deposited in Fermentation Research Institute, Ministry of International Trade and Industry, at 1-1-3, Higashi, Yatabe-cho, Tsukuba-gun, Ibaraki-ken, Japan on November 27, 1984 (deposit number FERM P-7957).

EXAMPLE 4

Stability of transformed E. coli K-12 YK2009:-

One hundred milliliters of the minimum medium indicated above was put in a 500 ml Erlenmeyer flask, and sterilized at 120°C for 15 minutes. Then, the transformants obtained in Example 3 were inoculated in the medium and cultivated with shaking at 37°C for 24 hours. One hundred milliliters of L medium was put in a 500 ml Erlenmeyer flask, and sterilized at 120°C for 15 minutes. The cultivated transformants were then inoculated in the L medium at a rate of 50 cells per ml and cultivated with shaking at 37°C for 24 hours. The cells were harvested by centrifugation, and washed. A predetermined amount of the cells was applied to a plate medium prepared as L medium containing 50 micrograms/ml of ampicillin and L medium containing no ampicillin, and cultivated at 37°C for 1 day. The number of grown colonies was counted.

The number of the grown colonies was the same for the two media, and all the colonies grown on the L medium grew on the minimum medium not containing tryptophan. The results demonstrate the high stability of the above plasmid.

EXAMPLE 5

Measurement of tryptophan synthase activity

One hundred milliliters of the minimum medium was put in a 500 ml Erlenmeyer flask, and sterilized at 120°C for 15 minutes. The transformants obtained in Example 1 were inoculated in the minimum medium, and cultivated with shaking at 37°C for 1 day. Then, 20 ml of the culture was inoculated in L medium containing indoleacrylic acid in a concentration of 100 microgarams/ml, and cultivated with shaking at 37°C for 6 hours. The culture broth was centrifuged to harvest the cells. The cells were washed with 50 ml of 100 mM Tris buffer (pH 7.8) and again centrifuged. Two hundred milligrams of wet cells were collected and suspended in 1 ml of 100 mM Tris buffer (pH 7.8). The suspension was subjected to ultrasonication. The resulting cell crushed product was diluted with 100 mM Tris buffer and subjected to an enzymatic reaction [O. H. Smith and C. Yanofsky: "Methods in Enzymology", Academic Press, New York (1962), vol. 5, pages 794-806]. It was found that when E. coli harboring plasmid pMTY-2 (FERM P-7838) was used, the synthase activity was about 100 units (1 unit = 0.1 micromole trp/mg protein/20 min.). When E. coli containing plasmid pMTY-3 (FERM P-7957) was used, the synthase activity was about 250 units.

EXAMPLE 6

Construction of plasmid pMTY-8

(A) Preparation of plasmid pMTY-3

One hundred milliliters of L medium (tryptone 10 g, yeast extract 5 g, NaCl 5 g, distilled water 1000 ml; pH 7.2) was put in a 500 ml Erlenmeyer flask, and sterilized at 120°C for 15 minutes. E. coli K-12 Y2009 (FERM P-7975) obtained in Example 4 was inoculated in the L medium, and cultivated at 37°C for 15 hours. Then, 20 ml of the culture broth was taken, and freshly inoculated in 100 ml of the above medium and again cultivated at 37°C for 5 hours.

After the cultivation, the entire culture broth was centrifuged (800 x g, 15 minutes, 4°C) to harvest the cells. The cells were treated with lysozyme, and lyzed with sodium dodecylsulfate (SDS). The lysate was centrifuged at 32,000 x g for 40 minutes. The supernatant was separated and then subjected to caesium chloride-ethidium bromide equilibrium density gradient centrifugation. Subsequent dialysis gave a solution containing plasmid pMTY-3. The solution was precipitated with ethanol, and finally about 100 micrograms of plasmid pMTY-3 was isolated.

(B) Re-ligation of DNA fragment containing genes governing the biosynthesis of tryptophan synthase

Five micrograms of the plasmid pMTY-3 prepared in (A) above was digested with restriction enzyme EcoRI at 37°C for 1 hour to cleave the plasmid pMTY-3 DNA. Then, the product was heat-treated at 60°C for 5 minutes to inactivate EcoRI. ATP and DTT were added, and DNA ligase (a product of Takara Shuzo Co., Ltd.) derived from T4 DNA ligase was also added. The mixture was maintained at 12°C for 17 hours, and then heat-treated at 60°C for 5 minutes to inactivate the DNA ligase. E. coli K-12 strain (tryptophan-auxotrophic mutant; ATCC 23718) was transformed with the ligated DNA to obtain transformants [the disappearance of tryptophan auxotrophy; they permit biosynthesis of tryptophan by trp A and trp B on the plasmid and can be grown on a mininum medium ($K_2HPO_4$ 7g, $KH_2PO_4$ 2g, $MgSO_4.7H_2O$) 0.1g, $(NH_4)_2SO_4$ 1g, glucose 2g, pure water 1 liter)]. The transformants were subjected to liquid cultivation, and a plasmid pMTY-8 having the restriction endonuclease map shown in Figure 4 of the attached drawings was isolated from the culture broth.

The transformants harboring the plasmid pMTY-8 were deposited in Fermentation Research Institute, Ministry of International Trade and Industry, at 1-1-3, Higashi, Yatabe-cho, Tsukuba-gun, Ibaraki-ken, Japan on July 5, 1985 (deposit number FERM P-8328).

The tryptophan synthase acivity of the resulting transformants, measured as in Example 5, was found to be about 300 units.

EXAMPLE 7

One hundred milliliters of the minimum medium was put in a 500 ml Erlenmeyer flask, and sterilized at 120°C for 15 minutes. The transformants E. coli K-12 YK2004 (FERM P7838) obtained in Example 1 were inoculated in the medium and cultivated with shaking at 37°C for 1 day. Then, 2 ml of the culture was inoculated in 50 ml of L medium put in a 500 ml Erlenmeyer flask and sterilized at 120°C for 15 minutes as above, and cultivated at 37°C for 5 hours. The cells were harvested by centrifugation, suspended in 50 ml of 100 mM Tris buffer (pH 7.8) containing 2.5 g of indole, 10.0 g of DL-serine and 0.5 g of pyridoxal-5'-phosphate, and reacted at 37°C for 24 hours with shaking. After the reaction, the reaction mixture was diluted to 10 times with water, and then centrifuged. The resulting supernatant was subjected to high-performance liquid chromatography, and it was found that 2.2 mg/ml of L-tryptophan was formed.

After the reaction, 500 ml of the diluted solution was passed through a column of an ammonia-type strongly acidic ion-exchange resin (Diaion® SK-1B, a product of Mitsuibishi Chemical) to precipitate crude crystals of L-tryptophan. The crude crystals were washed and dried to give 770 mg of L-tryptophan crystals.

EXAMPLE 8

One hundred milliliters of the minimum medium was put in a 500 ml Erlenmeyer flask, and sterilized at 120°C for 15 minutes. The transformants E. coli K-12 Y2004 (FERM P-7838) were inoculated and cultivated at 37°C for 1 day with shaking. Then, 2 ml of the culture was inoculated in 50 ml of L medium containing indoleacrylic acid in a concentration of 100 micrograms/ml in the same way as in Example 7, and cultivated with shaking at 37°C for 5 hours. The cells were harvested by centrifugation, and then reacted by the same operation as in Example 7. Analysis of the reaction product showed that L-tryptophan was formed in an amount of 3.1 mg/ml.

L-tryptophan was purified and recovered from 50 ml of the reaction solution after the reaction by the

EP 0 180 192 B1

same operation as in Example 7. There was obtained 1.0 g of L-tryptophan as crystals.

EXAMPLE 9

Fifty milliliters of a minimum medium having the following composition was put in a 500 ml Erlenmeyer flask, and sterilized at 120°C for 15 minutes. The transformants E. coli K-12 YK2009 (FERM P-7957) obtained in Example 4 wre inoculated in the medium and cultivated with shaking at 37°C for 1 day. Then, 2 ml of the culture was inoculated in 50 ml of L medium having the composition indicatd above put in a 500 ml Erlenmeyer flask and sterilized at 120°C for 15 minutes, and cultivated with shaking at 37°C for 5 hours.

<u>Composition of the minimum medium</u>

| | |
|---|---|
| $K_2HPO_4$ | 7.0 g |
| $KH_2PO_4$ | 2.0 g |
| $(NH_4)_2SO_4$ | 0.1 g |
| Glucose | 2.0 g |
| Water | 1 liter |
| pH | not adjusted |

The cells were recovered by centrifugation, suspended in 50 ml of 100 mM Tris buffer (pH 7.8) containing 2.5 g of indole, 10.0 g of DL-serine and 0.5 mg of pyridoxal-5'- phosphate, and reacted at 37°C for 15 hours with shaking. After the reaction, the reaction mixture was diluted to 10 times, and centrifuged. The supernatant was analyzed by high-performance liquid chromatography. It was found that L-tryptophan was formed in an amount of 3.5 mg/ml.

After the reaction, 500 ml of the 10-fold dilution was passed through a column of an ammonia-type strongly acidic ion-exchange resin (Diaion® SK-1B, a product of Mitsubishi Chemical Co., Ltd.) to adsorb L-tryptophan. The column was eluted with an alkaline solution and then concentrated to precipitate crude crystals of L-tryptophan. The crude crystals were washed with acetone and dried to obtain 1.2 g of L-tryptophan crystals.

EXAMPLE 10

Fifty milliliters of the minimum medium was put in a 500 ml Erlenmeyer flask, and sterilized at 120°C for 15 minutes. The transformants E. coli K-12 YK2009 (FERM-P 7957) were inoculated in the minimum medium and cultivated at 37°C for 1 day. Two milliliters of the culture was inoculated in the same way as in Example 9 in 50 ml of L medium containing indoleacrylic acid in a concentration of 100 micrograms/ml, and cultivated with shaking at 37°C for 5 hours. The cells were recovered by centrifugation, and then reacted in the same way as in Example 9. After the reaction, the reaction mixture was analyzed in the same way as in Example 9. It was found that L-tryptophan was formed in an amount of 6.0 mg/ml.

L-tryptophan was purified and recovered from 50 ml of the reaction mixture by the same operation as in Example 9. There was obtained 2.1 g of L-tryptophan as crystals.

EXAMPLE 11

Ten milliliters of L medium having the same composition as described in Example 1, (A) was put in a large-sized test tube (200 x 24 φ) and sterilized at 120°C for 15 minutes. Then, E. coli K-12 YK2004 (FERM P-7838) was inoculated, and cultivated at 37°C for 15 hours to form a preculture. Two milliliters of the preculture was inoculated in a medium A of the following composition (put in an amount of 100 ml in a 500 ml Erlenmeyer flask) containing 100 micrograms/ml of indoleacrylic acid and 0.5 mg/ml of indole, and cultivated with shaking at 37°C for 48 hours.

14

Composition of the culture medium A

| | |
|---|---|
| $NH_4Cl$ | 4 g |
| $KH_2PO_4$ | 3 g |
| $Na_2HPO_4 \cdot 12H_2O$ | 15 g |
| $MgSO_4 \cdot 7H_2O$ | 0.5 g |
| $CaCl_2 \cdot 2H_2O$ | 15 mg |
| Casamino acid | 5 g |
| Yeast extract | 5 g |
| Glucose | 30 g |
| $CaCO_3$ (separately sterilized) | 20 g |
| Water | 1 liter |
| pH | 7.0 |

After the cultivation, the cells were removed by centrifugation, and L-tryptophan in the supernatant was quantitatively determined by high-performance liquid chromatography (LC-5A, a product of Shimadzu Seisakusho Co., Ltd.). It was found that L-tryptophan was formed in an amount of 80 mg/liter.

As a control, the same operation as above was carried out except that E. coli K-12 transformed with a plasmid resulting from the removal of the mini-F fragment from the plasmid pMTY-2. The amount of L-tryptophan formed was 10 mg/liter.

EXAMPLE 12

E. coli K-12 YK2004 (FERM P-7838) was pre-cultivated by the same operation as in Example 1. Two milliliters of the pre-culture so obtained was inoculated in the medium A containing 100 micrograms/ml of indoleacrylic acid and 1 mg/ml of anthranilic acid (put in an amount of 100 ml in a 500 ml Erlenmeyer flask), and cultivated with shaking at 37° C for 48 hours.

After the cultivation, the culture broth was centrifuged, and L-tryptophan in the supernatant was quantitatively determined. It was found that L-tryptophan was formed in an amount of 25 mg/liter.

When the same control experiment as in Example 11 was carried out, the amount of L-tryptophan was 5 mg/liter.

The separation and purification of L-tryptophan from the culture broth can be carried out by passing the supernatant of the culture broth after removing the cells through a column filled with activated carbon or an ammonia-type strongly acidic ion exchange resin to adsorb L-tryptophan. The column is fully washed with water and then eluted with an ammonia solution. The eluates were concentrated, washed with acetone and dried to precipitate L-tryptophan crystals.

EXAMPLE 13

Ten milliliters of L medium (tryptone 10 g, yeast extract 5 g, NaCl 5 g, glucose 1 g, water 1 liter; pH 7.2) was put in a large-sized test tube (200 x 24 $\phi$), and sterilized at 120° C for 15 minutes. The E. coli K-12 YK2009 (FERM P-7957) obtained in Example 3 was inoculated in the L medium, and cultivated with shaking at 37° C for 16 hours to form a preculture. Two milliliters of the pre-culture was inoculated in a culture medium A having the above composition containing 100 micrograms/ml of indoleacrylic acid and 0.5 mg/ml of indole (put in an amount of 100 ml in a 500 ml Erlenmeyer flask), and cultivated with shaking for 48 hours.

After the cultivation, the cells were removed by centrifugation, and L-tryptophan in the supernatant was quantitatively determined by high-performance liquid chromatography (LC-5A made by Shimadzu Seisakusho Co., Ltd.). It was found that L-tryptophan was formed in an amount of 100 mg/liter.

As a control, the same operation as above was carried out using E. coli K-12 transformed with a plasmid resulting from the removal of the mini-F fragment from the plasmid pMTY-3. The amount of L-tryptophan formed was 20 mg/liter.

EXAMPLE 14

One hundred milliliters of the minimum medium was put in a 500 ml Erlenmeyer flask, and sterilized at 120°C for 15 minutes. The transformatns E. coli K-12 YK2014 (FERM P-8328) were inoculated in the minimum medium, and cultivated at 37°C for 1 day. Fifty milliliters of the L medium was put in an Erlenmeyer flask, and sterilized at 120°C for 15 minutes. Two milliliters of the above culture was inoculated in the L medium, and cultivted at 37°C for 5 hours. The cells were harvested by centrifugation, suspended in 50 ml of 100 mM Tris buffer (pH 7.8) containing 2.5 g of indole, 10.0 g of DL-serine and 0.5 ml of pyridoxal-5'- phosphate, and reacted with shaking at 37°C for 24 hours. The reaction mixture was then diluted to 10 times with water, and centrifuged. The supernanant was analyzed by high-performance liquid chromatography. It was found that L-tryptophan was formed in an amount of 4.0 mg/ml.

500 ml of the 10-fold dilution of the reaction mixture was passed through a column of an ammonia-type strongly acidic ion-exchange resin (Diaion® SK-1B,a product of Mitsubishi Chemical Co., Ltd.) to precipitate crude crystals of L-tryptophan. The crude crystals were washed with acetone, and dried to give 1.4 g of L-tryptophan crystals.

EXAMPLE 15

One hundred milliliters of the minimum medium was put in a 500 ml Erlenmeyer flask, and sterilized at 120°C for 15 minutes. The transformants E. coli K-12 YK2014 (FERM P-8328) were inoculated in the minimum medium, and cultivated with shaking at 37°C for 1 day. Two milliliters of the culture was inoculated in the same way as in Example 14 in 50 ml of L-medium containing indole acrylic acid in a concentration of 100 micrograms/ml, and cultivated with shaking at 37°C for 5 hours. The cells were recovered by centrifugation, and reacted by the same operation as in Example 14. It was found that 7 mg/ml of L-tryptophan was obtained.

After the reaction, 50 ml of the reaction mixture was worked up in the same way as in Example 14 to purify and recover L-tryptophan. There was obtained 2.3 g of L-tryptophan as crystals.

EXAMPLE 16

Ten milliliters of L medium having the same composition as indicated in Example 1, (A) was put in a large-sized test tube (200 x 24 φ), and sterilized at 120°C for 15 minutes. E. coli K-12 YK2014 (FERM P-8328) was inoculated in the L medium and cultivated at 37°C for 16 hours. Two milliliters of the culture was inoculated in a culture medium A having the same composition as indicated in Example 11 containing 100 micrograms/ml of indoleacrylic acid and 0.5 mg/ml of indole (put in an amount of 100 ml in a 500 ml Erlenmeyer flask), and cultivated at 37°C for 48 hours.

After the cultivation, the cells were removed by centrifugation, and the supernatant was analyzed by high-performance liquid chromatography (LC-5A produced by Shimadzu Seisakusho Co., Ltd.). It was found that L-tryptophan was formed in an amount fo 120 mg/liter.

**Claims**

**1.** A plasmid which can be autonomously replicated within the cells of Escherichia coli, in which plasmid
(a) a DNA fragment containing genes which govern the biosynthesis of tryptophan synthase consists at least of trp A, trp B, a DNA fragment having a promoter function of expressing said two genes, and a DNA fragment having an operator function of controlling said promoter function,
(b) a DNA fragment containing genes which are derived from F-plasmid and govern its partition system is a mini-F fragment,
(c) a DNA fragment containing genes which are derived from the Col El-type plasmid and govern its autonomous replication is derived from plasmid pBR322,
(i) the direction of transcription from the promoter in the DNA fragment containing genes governing the biosynthesis of tryptophan synthase, the direction of replication from the replication initiation point of the mini-F fragment, and the direction of replication from the replication initiation point of the DNA fragment containing genes which are derived from Col El-type plasmid and govern its autonomous replication are identical, and

(ii) the DNA fragment containing genes governing the biosynthesis of tryptophan synthase is located upstream of the DNA fragment containing genes which are derived from the Col El-type plasmid and govern its autonomous replication.

2. The plasmid of claim 1 which is plasmid pMTY-8 according to Figure 4 having a molecular size of about 9.0 megadaltons, and 2, 2 and 3 sites of cleavage by restriction endonucleases EcoRI, BamHI and SalI, and in which the BamHI fragments have a molecular size of about 6.4 megadaltons and about 2.6 megadaltons.

3. A plasmid which is plasmid pMTY-2 according to Figure 1 having a molecular size of about 13.7 megadaltons and 2, 3 and 2 sites of recognition by restriction endonucleases EcoRI, BamHI and SalI, respectively.

4. A plasmid which is plasmid pMTY-3 according to Figure 3 having a molecular size of about 9.0 megadaltons and 2, 2 and 3 sites of recognition by restriction endonucleases EcoRI, BamHI and SalI, respectively.

5. A microorganism of the E. coli K-12 type which is transformed with plasmid pMTY-2.

6. A microorganism of the E. coli K-12 type which is transformed with plasmid pMTY-3.

7. A microorganism of the E. coli K-12 type which is transformed with plasmid pMTY-8.

8. The microorganism of anyone of claims 1 to 7 wherein the microorganism is a tryptophanase-deficient mutant.

9. A process for producing L-tryptophan, which comprises reacting indole with L- or DL-serine in the presence of a cultivated product of a microorganism of the genus Escherichia transformed with the plasmid of claim 1, or a treated product of the cultivation product.

10. The process of claim 9 wherein the microorganism of the genus Escherichia is a strain not having the L-tryptophan decomposing activity.

11. The process of claim 9 wherein the cultivated product is obtained by cultivating the microorganism in a culture medium containing indoleacrylic acid or its salt.

12. The process of claim 11 wherein indoleacrylic acid or its salt is added in a final concentration of at least 25 micrograms/ml before the final stage of the logarithmic growth period of the microorganism.

13. A process for producing L-tryptophan, which comprises cultivating a microorganism of the genus Escherichia transformed with the plasmid of claim 1 in a culture medium, and recovering L-tryptophan from the culture broth.

14. The process of claim 13 wherein indoleacrylic acid or its salt is added to the culture medium.

15. The process of claim 14 wherein indoleacrylic acid or its salt is added to the culture medium in a final concentration of at least 25 micrograms/ml before the final stage of the logarithmic growth period of the microorganism at the latest.

16. The process of claim 13 wherein indole or anthranilic acid or its salt is added further to the culture medium.

**Revendications**

1. Plasmide qui peut être répliqué de façon autonome dans les cellules de *Escherichia coli*, dans lequel
   (a) un fragment d'ADN contenant des gènes qui commandent la biosynthèse de la tryptophane-synthétase est constitué au moins par trp A, trp B, un fragment d'ADN ayant une fonction de promoteur pour l expression de ces deux gènes, et un fragment d'ADN ayant une fonction d'opérateur pour réguler ladite fonction de promoteur,

(b) un fragment d'ADN contenant des gènes qui sont dérivés du plasmide F et commandent son système de partage est un mini-fragment F,

(c) un fragment d'ADN contenant des gènes qui sont dérivés du plasmide de type Col E1 et commandent sa réplication autonome est dérivé du plasmide pBR322,

(i) la direction de transcription à partir du promoteur dans le fragment d'ADN contenant des gènes qui commandent la biosynthèse de la tryptophane-synthétase, la direction de réplication à partir du point d'initiation de réplication du mini-fragment F, et la direction de réplication à partir du point d'initiation de réplication du fragment d'ADN contenant des gènes qui sont dérivés du plasmide de type Col E1 et commandent sa réplication autonome sont identiques, et

(ii) le fragment d'ADN contenant des gènes qui commandent la biosynthèse de la tryptophane-synthétase est situé en amont du fragment d'ADN contenant des gènes qui sont dérivés du plasmide de type Col E1 et commandent sa réplication autonome.

2. Plasmide selon la revendication 1, qui est le plasmide pMTY-8 selon la Figure 4 ayant une taille moléculaire d'environ 9,0 mégadaltons et comportant 2, 2 et 3 sites de clivage par les endonucléases de restriction *Eco*RI, *Bam*HI et *Sal*I, et dans lequel les fragments *Bam*HI ont une taille moléculaire d'environ 6,4 mégadaltons et d'environ 2,6 mégadaltons.

3. Plasmide qui est le plasmide pMTY-2 selon la Figure 1 ayant une taille moléculaire d'environ 13,7 mégadaltons et comportant 2, 3 et 2 sites de reconnaissance par les endonucléases de restriction *Eco*RI, *Bam*HI et *Sal*I, respectivement.

4. Plasmide qui est le plasmide pMTY-3 selon la Figure 3 ayant une taille moléculaire d'environ 9,0 mégadaltons et comportant 2, 2 et 3 sites de reconnaissance par les endonucléases de restriction *Eco*RI, *Bam*HI et *Sal*I, respectivement.

5. Micro-organisme du type *E. coli* K-12 qui est transformé par le plasmide pMTY-2.

6. Micro-organisme du type *E. coli* K-12 qui est transformé par le plasmide pMTY-3.

7. Micro-organisme du type *E. coli* K-12 qui est transformé par le plasmide pMTY-8.

8. Micro-organisme selon l'une quelconque des revendications 1 à 7, dans lequel le micro-organisme est un mutant déficient en tryptophanase.

9. Procédé de production du L-tryptophane, qui consiste à faire réagir de l'indole avec la L- ou DL-sérine en présence d un produit de culture d'un micro-organisme du genre *Escherichia* transformé par le plasmide de la revendication 1, ou d'un produit de traitement du produit de culture.

10. Procédé selon la revendication 9, dans lequel le micro-organisme du genre *Escherichia* est une souche ne possédant pas l'activité de décomposition du L-tryptophane.

11. Procédé selon la revendication 9, dans lequel le produit de culture est obtenu en cultivant le microorganisme dans un milieu de culture contenant de l'acide indole-acrylique ou un sel de celui-ci.

12. Procédé selon la revendication 11, dans lequel l'acide indole-acrylique ou son sel est ajouté à une concentration finale d'au moins 25 microgrammes/ml avant le dernier stade de la phase de croissance exponentielle du micro-organisme.

13. Procédé de production du L-tryptophane, qui consiste à cultiver un micro-organisme du genre *Escherichia* transformé par le plasmide de la revendication 1 dans un milieu de culture, et à retirer le L-tryptophane du bouillon de culture.

14. Procédé selon la revendication 13, dans lequel on ajoute de l'acide indole-acrylique ou un sel de celui-ci au milieu de culture.

**15.** Procédé selon la revendication 14, dans lequel on ajoute l'acide indole-acrylique ou son sel au milieu de culture à une concentration finale d'au moins 25 microgrammes/ml au plus tard avant le dernier stade de la phase de croissance exponentielle du micro-organisme.

**16.** Procédé selon la revendication 13, dans lequel on ajoute de plus, au milieu de culture, de l'indole, de l'acide anthranilique ou un sel de celui-ci.

## Patentansprüche

**1.** Plasmid, das innerhalb der Zellen von Escherichia coli autonom repliziert werden kann, dadurch **gekennzeichnet**, daß in dem Plasmid

(a) ein DNA-Fragment, das Gene enthält, die die Biosynthese der Tryptophan-Synthase steuern, aus mindestens trp A, trp B, einem DNA-Fragment mit einer Promotor-Funktion zur Expression der zwei Gene und einem DNA-Fragment mit einer Operator-Funktion zur Kontrolle der Promotor-Funktion besteht,

(b) ein DNA-Fragment, das Gene enthält, die sich von dem F-Plasmid ableiten und dessen Verteilungssystem dirigieren, ein Mini-F-Fragment ist,

(c) ein DNA-Fragment, das Gene enthält, die sich von dem Col-E1-Typ-Plasmid ableiten und dessen autonome Replikation dirigieren, sich von dem Plasmid pBR322 ableitet, wobei

(i) die Transkriptionsrichtung von dem Promotor in dem DNA-Fragment, das Gene enthält, die die Biosynthese der Tryptophan-Synthase dirigieren, die Replikationsrichtung von dem Replikationsstartpunkt des Mini-F-Fragments und die Replikationsrichtung von dem Replikationsstartpunkt des DNA-Fragments, das Gene enthält, die sich von dem Col-E1-Typ-Plasmid ableiten und dessen autonome Replikation dirigieren, identisch sind, und

(ii) das DNA-Fragment, das Gene enthält, die die Biosynthese der Tryptophan-Synthase dirigieren, stromaufwärts von dem DNA-Fragment, welches Gene enthält, die sich von dem Col-E1-Typ-Plasmid ableiten und dessen autonome Replikation dirigieren, angebracht ist.

**2.** Plasmid nach Anspruch 1, dadurch **gekennzeichnet**, daß das Plasmid pMTY-8 gemäß Figur 4 ist und eine Molekulgröße von etwa 9,0 Megadalton und 2, 2 und 3 Restriktionsspaltstellen durch die Endonukleasen EcoRI, BamHI und SalI besitzt und worin die BamHI-Fragmente eine Molekülgröße von etwa 6,4 Megadalton und etwa 2,6 Megadalton besitzen.

**3.** Plasmid, welches Plasmid pMTY-2 gemäß Figur 1 ist, und welches eine Molekülgröße von etwa 13,7 Megadalton und 2, 3 und 2 Erkennungsstellen für die Restriktions-Endonukleasen EcoRI, BamHI bzw. SalI besitzt.

**4.** Plasmid, welches Plasmid pMTY-3 gemäß Figur 3 ist, und welches eine Molekülgröße von etwa 9,0 Megadalton und 2, 2 und 3 Erkennungsstellen für die Restriktions-Endonukleasen EcoRI, BamHI bzw. SalI besitzt.

**5.** Mikroorganismus vom E. coli K-12-Typ, welcher mit dem Plasmid pMTY-2 transformiert worden ist.

**6.** Mikroorganismus vom E. coli K-12-Typ, welcher mit dem Plasmid pMTY-3 transformiert worden ist.

**7.** Mikroorganismus vom E. coli K-12-Typ, welcher mit dem Plasmid pMTY-8 transformiert worden ist.

**8.** Mikroorganismus nach einem der Ansprüche 1 bis 7, dadurch **gekennzeichnet**, daß der Mikroorganismus eine Tryptophanase-defiziente Mutante ist.

**9.** Verfahren zur Herstellung von L-Tryptophan, dadurch **gekennzeichnet**, daß man Indol mit L- oder DL-Serin in Gegenwart eines Kulturprodukts eines Mikroorganismus der Gattung Escherichia, der mit dem Plasmid nach Anspruch 1 oder einem behandelten Produkt des Kulturprodukts transformiert worden ist, umsetzt.

**10.** Verfahren nach Anspruch 9, dadurch **gekennzeichnet**, daß der Mikroorganismus der Gattung Escherichia ein Stamm ist, der die Aktivität, L-Tryptophan zu zersetzen, nicht besitzt.

**11.** Verfahren nach Anspruch 9, dadurch **gekennzeichnet**, daß das Züchtungsprodukt dadurch erhalten wird, daß man den Mikroorganismus in einem Kulturmedium, das Indolacrylsäure oder deren Salz enthält, züchtet.

**12.** Verfahren nach Anspruch 11, dadurch **gekennzeichnet**, daß die Indolacrylsäure oder deren Salz in einer Endkonzentration von mindestens 25 Mikrogramm/ml vor der Endstufe der logarithmischen Wachstumsperiode des Mikroorganismus zugesetzt wird.

**13.** Verfahren zur Herstellung von L-Tryptophan, dadurch **gekennzeichnet**, daß man einen Mikroorganismus der Gattung Escherichia, der mit dem Plasmid nach Anspruch 1 transformiert worden ist, in einem Kulturmedium kultiviert und L-Tryptophan aus der Kulturlösung gewinnt.

**14.** Verfahren nach Anspruch 13, dadurch **gekennzeichnet**, daß Indolacrylsäure oder deren Salz dem Kulturmedium zugesetzt wird.

**15.** Verfahren nach Anspruch 14, dadurch **gekennzeichnet**, daß Indolacrylsäure oder deren Salz dem Kulturmedium in einer Endkonzentration von mindestens 25 Mikrogramm/ml vor der Endstufe der logarithmischen Wachstumsperiode des Mikroorganismus spätestens zugesetzt wird.

**16.** Verfahren nach Anspruch 13, dadurch **gekennzeichnet**, daß weiterhin Indol oder Anthranilsäure oder deren Salz dem Kulturmedium zugesetzt werden.

FIG. 1

FIG. 4

FIG. 2

FIG. 3